(19) Europäisches Patentamt / European Patent Office / Office européen des brevets

(11) **EP 3 071 098 B1**

(12) **EUROPÄISCHE PATENTSCHRIFT**

(45) Veröffentlichungstag und Bekanntmachung des Hinweises auf die Patenterteilung:
**27.09.2017 Patentblatt 2017/39**

(21) Anmeldenummer: **15807651.3**

(22) Anmeldetag: **08.12.2015**

(51) Int Cl.:
*A61B 5/021* *(2006.01)*      *A61B 5/0225* *(2006.01)*
*A61B 5/00* *(2006.01)*

(86) Internationale Anmeldenummer:
**PCT/EP2015/078899**

(87) Internationale Veröffentlichungsnummer:
**WO 2016/091839 (16.06.2016 Gazette 2016/24)**

(54) **VERFAHREN UND GERÄT ZUR BESTIMMUNG MINDESTENS EINES PHYSIOLOGISCHEN PARAMETERS MITTELS KORRIGIERTEM PULSMESSSIGNAL**

METHOD AND DEVICE FOR DETERMINING AT LEAST ONE PHYSIOLOGICAL PARAMETER USING A CORRECTED PULSE MEASUREMENT SIGNAL

PROCÉDÉ ET APPAREIL POUR DÉTERMINER AU MOINS UN PARAMÈTRE PHYSIOLOGIQUE AU MOYEN D'UN SIGNAL DE MESURE DU POULS CORRIGÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **10.12.2014 DE 102014225483**

(43) Veröffentlichungstag der Anmeldung:
**28.09.2016 Patentblatt 2016/39**

(73) Patentinhaber: **Küchler, Gert**
**97236 Randersacker (DE)**

(72) Erfinder:
• **MÖRSDORF, Hans-Joachim**
**90530 Wendelstein (DE)**
• **KÜCHLER, Gert**
**97236 Randersacker (DE)**

(74) Vertreter: **Rau, Schneck & Hübner**
**Patentanwälte Rechtsanwälte PartGmbB**
**Königstraße 2**
**90402 Nürnberg (DE)**

(56) Entgegenhaltungen:
**US-A1- 2003 036 685    US-A1- 2004 171 944**
**US-A1- 2005 154 299**

• **Y.-T. SHIH ET AL: "Application of the N-Point Moving Average Method for Brachial Pressure Waveform-Derived Estimation of Central Aortic Systolic Pressure", HYPERTENSION, Bd. 63, Nr. 4, 1. April 2014 (2014-04-01), Seiten 865-870, XP055202082, ISSN: 0194-911X, DOI: 10.1161/HYPERTENSIONAHA.113.02229**

**Beschreibung**

Die Erfindung betrifft Verfahren und ein Gerät zur Bestimmung mindestens eines physiologischen Parameters eines Patienten.

Die Erfassung physiologischer Parameter ist in der Medizintechnik heutzutage üblich und weit verbreitet. Ein Beispiel für eine derartige Erfassung eines physiologischen Parameters ist die kontinuierliche Messung des arteriellen Blutdrucks. Ein derartiges Gerät und zugehöriges Erfassungsverfahren, die ohne die bekannte aufpumpbare Armmanschette mit dem nach dem Riva-Rocci-Prinzip funktionierenden Drucksensor auskommt, sind in der DE 10 2005 014 048 B4 beschrieben. Das Erfassungsverfahren basiert auf der Auswertung der Pulsdruckwellenlaufzeit (Pulse Transit Time = PTT). Dabei wird die Laufzeit der Pulsdruckwelle vom Herzen bis in die Peripherie, beispielsweise bis an einen der Finger, für jeden Herzschlag bestimmt. Als Startzeitpunkt der Laufzeitmessung dient die R-Zacke des EKGs und als Endzeitpunkt der Zeitpunkt, an dem das an der Peripherie, also z.B. an dem Finger, insbesondere photoplethysmographisch oder pulsoximetrisch erfasste Pulsmesssignal die maximale Steigung aufweist. Aus der so ermittelten Laufzeit wird unter Berücksichtigung weiterer Parameter, wie beispielsweise der Körpergröße, die Pulswellengeschwindigkeit (= PWG) und daraus der letztendlich interessierende Blutdruck ermittelt. Dieses Blutdruckmessgerät hat sich in der Praxis bewährt. Es funktioniert in den meisten Anwendungsfällen sehr gut. Allerdings treten bisweilen Messfehler auf, so dass ein Verbesserungsbedarf besteht.

Weiterhin wird in der DE 10 2007 024 072 A1 ein Verfahren zur Darstellung und Auswertung von EKG-Signalen und atmungsabhängigen Signalen beschrieben. Erfasst werden physiologische Parameter wie der Blutdruck und die Sauerstoffsättigung, letztere mittels eines Pulsoximeters. Um störende Artefakte, wie z.B. ein mögliches 50 Hz-Netzbrummen, zu entfernen, wird ein 50 Hz-Filter eingesetzt. Außerdem kommt ein Bandpassfilter zum Einsatz, das irrelevante Frequenzanteile des erfassten Pulsmessignals ausblendet.

In der DE 689 25 988 T2 wird ein Verfahren zur Kompensation von Verzerrungen in einem Pulsoximeter beschrieben. Die Verzerrungen können durch Artefakte infolge lokaler Änderungen des Blutvolumens, durch transiente Sättigung oder durch Blutvolumenartefakte verursacht sein. Die Kompensation erfolgt zumindest teilweise mittels einer Frequenzfilterung.

In der DE 601 30 395 T2 wird eine Vorrichtung zum Überwachen des Fortschreitens des Krankheitszustandes eines Patienten mit Herzinsuffizienz beschrieben. Die Vorrichtung umfasst Mittel zum Erfassen eines physiologischen Signals, das ein Indikator für eine Amplitude eines arteriellen Pulses des Patienten ist. Unter anderem wird dabei auch ein Pulsmesssignal pulsoximetrisch erfasst und einer Breitband- und Schmalbandfilterung unterzogen.

In der DE 60 2004 000 513 T2 wird ein System zur Spektroskopieanalyse von Blutkomponenten beschrieben. Das Analysesystem umfasst einen Signalprozessor, der ein photo-pethysmografisches Signal entsprechend einer speziellen Wellenlänge aus einem elektrischen Signal extrahiert. Das System umfasst eine Verstärker- und Filtereinheit, die aus einem verstärkten elektrischen Signal eine Rauschkomponente eliminiert.

In der DE 10 2006 022 120 A1 wird ein Signalverarbeitungsverfahren beschrieben, das in plethysmogrammbasierten Messverfahren zum Zwecke einer niedrigen Störanfälligkeit bei Umgebungslichtinterferenzen und elektromagnetischen Interferenzen Anwendung findet. Das Signalverarbeitungsverfahren umfasst auch verschiedene Frequenzfilter, die aber in enger Verbindung zu der verwendeten speziellen Spreizspektrummodulation/-demodulation stehen.

In der DE 198 29 544 C1 wird eine Vorrichtung zur nichtinvasiven Blutdruckmessung beschrieben. Mittels Ultraschall- oder Laser-Dopplertechnik wird z.B. eine mit dem Blutfluss oder der Blutflussgeschwindigkeit verknüpfte Größe gemessen. Die der Messwerterfassung nachgeschaltete Signalverarbeitung umfasst auch eine Filterung zur Beseitigung von Artefakten und sonstigen Störungen.

In der EP 2 491 856 A1 werden ein Verfahren und ein Gerät zur Pulsdetektion beschrieben, wobei ein auf eine Körperbewegung zurückzuführender Rauschanteil in dem erfassten Pulsmesssignal mittels adaptiver Filterung eliminiert wird. Hierzu ist unter anderem auch ein gesonderter Sensor vorgesehen, der die Körperbewegung erfasst.

In der US 2014/0 288 445 A1 werden ein Verfahren und ein Gerät zur Blutdruckerfassung beschrieben. Es wird eine reflektierte Welle erfasst und zur Validierung eines erfassten Pulssignals verwendet.

In der DE 698 35 843 T2 werden ein weiteres Verfahren und ein weiteres Gerät zur Pulswellenuntersuchung beschrieben.

In der US 2004/171944 A1 werden ein Verfahren und ein Gerät zur Bestimmung einer Pulswellenausbreitungsgeschwindigkeit eines Patienten beschrieben, bei dem ein Pulsmesssignal einer Pulsdruckwelle, die sich ausgehend vom Herzen innerhalb der Blutgefäße ausbreitet, am Arm des Patienten erfasst wird, aus dem erfassten Pulsmesssignal mittels Signalverarbeitung ein korrigiertes Pulsmesssignal erzeugt wird, wobei das erfasste Pulsmesssignal einer Filterung unterzogen wird, um den Einfluss eines reflektierten Anteils der Pulsdruckwelle zu kompensieren, und die Pulswellenausbreitungsgeschwindigkeit anhand des korrigierten Pulsmesssignals ermittelt wird.

In der US 2003/0036685 A1 wird ein Überwachungssystem zur Erfassung physiologischer Signale beschrieben. Mittels eines photoplethysmographischen Sensors wird ein Pulswellensignal an einer Körperextremität erfasst. Aus den Messwerten werden nach einer Datenverarbeitung, bei der verschiedene Algorithmen, wie beispielsweise eine Bandpassfilterung, zum Einsatz kommen können, physiologische Parameter, wie z. B. der Blutdruckmittelwert, die Herzfrequenz, die Körpertemperatur, die Atemrate und der Blutdruck, abgeleitet. Weiterhin kann die sich in der Aorta ausbreitende reflektierte Pulswelle durch konventionelle adaptive Filter-Techniken ermittelt werden.

Eine Aufgabe der Erfindung besteht nun darin, ein Verfahren der eingangs bezeichneten Art mit einer gegenüber dem Stand der Technik verbesserten Erfassungsqualität anzugeben.

Zur Lösung dieser Aufgabe wird .ein Verfahren entsprechend den Merkmalen des Patentanspruchs 1 angegeben. Bei dem erfindungsgemäßen Verfahren handelt es sich um ein solches, bei dem ein Pulsmesssignal einer Pulsdruckwelle, die sich ausgehend vom Herzen innerhalb der Blutgefäße ausbreitet, an einer Pulsmessstelle erfasst wird, aus dem erfassten Pulsmesssignal mittels Signalverarbeitung ein korrigiertes Pulsmesssignal erzeugt wird, und der mindestens eine physiologische Parameter anhand des korrigierten Pulsmesssignals ermittelt wird. Zur Erzeugung des korrigierten Pulsmesssignals wird das erfasste Pulsmesssignal einer adaptiven Filterung mit einer sich dynamisch anpassenden Filtercharakteristik unterzogen, um den Einfluss eines reflektierten Anteils der Pulsdruckwelle zu kompensieren. Das erfasste Pulsmesssignal wird in jeweils einem Herzschlag zuzuordnende Messabschnitte zerlegt, aus jedem Messabschitt wird mittels adaptiver Filterung ein korrigierter Abschnitt ermittelt und die so erzeugten korrigierten Abschnitte werden zu dem korrigierten Pulsmesssignal zusammengesetzt.

Bei dem Pulsmesssignal der Pulsdruckwelle kann es sich insbesondere um ein Plethysmogramm handeln.

Es wurde erkannt, dass durch eine adaptive Filterung mit einer sich dynamisch anpassenden Filtercharakteristik Messfehler unterbunden werden können. Solche Fehler können ansonsten insbesondere aufgrund des Einflusses der reflektierten (oder rücklaufenden) Pulsdruckwelle auftreten. Dieser negative Einfluss der reflektierten Pulsdruckwelle lässt sich besonders effizient kompensieren, wenn keine starre Filterung, sondern eine adaptive Filterung mit dynamischer Anpassung der Filtercharakteristik zur Anwendung kommt. Dabei kann die dynamische Anpassung der Filtercharakteristik der adaptiven Filterung insbesondere an physiologische Gegebenheiten des Patienten und/oder vorzugsweise an den insbesondere auch durch z.B. kurzfristige Aktivierungen des autonomen Nervensystems beeinflussten aktuellen Gefäßzustand erfolgen.

Dadurch, dass das erfasste Pulsmesssignal in jeweils einem Herzschlag zuzuordnende Messabschnitte zerlegt und so weiter bearbeitet wird, ist eine sehr genaue Korrektur des erfassten Pulsmesssignals möglich. Die Korrektur ist dann insbesondere an die im jeweiligen Messabschnitt herrschenden Gegebenheiten angepasst. Es wurde erkannt, dass sich diese Gegebenheiten durchaus von Messabschnitt zu Messabschnitt verändern können, so dass eine messabschnittsweise erfolgende Signalkorrektur vorteilhaft ist.

Vorteilhafte Ausgestaltungen des erfindungsgemäßen Verfahrens ergeben sich aus den Merkmalen der von Anspruch 1 abhängigen Ansprüche.

Gemäß einer günstigen Ausgestaltung wird der betreffende Messabschnitt des erfassten Pulsmesssignals (PM) mittels Transformation in den Frequenzbereich in ein anfängliches Frequenzsignal überführt. Weiterhin wird das anfängliche Frequenzsignal zur Anpassung der Filtercharakteristik herangezogen und dann der adaptiven Filterung mit der angepassten Filtercharakteristik unterzogen, wobei ein korrigiertes Frequenzsignal gebildet wird, das mittels Rücktransformation in den Zeitbereich in den korrigierten Abschnitt überführt wird. Im Frequenzbereich lassen sich durch die reflektierte Pulsdruckwelle verursachte störende Signalanteile sehr effizient extrahieren und ausblenden, wobei die Frequenzfilterung vorteilhafterweise an die jeweils herrschenden und insbesondere anhand des erfassten Pulsmesssignals bzw. des daraus gewonnen anfänglichen Frequenzsignals erkannten Gegebenheiten angepasst wird.

Gemäß einer weiteren günstigen Ausgestaltung wird die adaptive Filterung als adaptive Tiefpassfilterung mit einer variablen Tiefpass-Grenzfrequenz durchgeführt. Diese Art der Filterung hat sich als sehr effizient erwiesen.

Gemäß einer weiteren günstigen Ausgestaltung wird die adaptive Filterung als adaptive Tiefpassfilterung mit einer variablen Tiefpass-Grenzfrequenz ausgeführt und werden von dem anfänglichen Frequenzsignal die Amplitudenmaxima bestimmt. Weiterhin wird anhand des Quotienten des zweiten Amplitudenmaximums zu dem dritten Amplitudenmaximum, also insbesondere

anhand des Quotienten $\dfrac{2.Maximum}{3.Maximum}$, der aktuelle Wert der Tiefpass-Grenzfrequenz ermittelt, um die Filtercharakteristik anzupassen. Es wurde erkannt, dass das zweite und dritte Amplitudenmaximum des anfänglichen Frequenzsignals sehr gut als Maß für die aktuell herrschenden Gegebenheiten insbesondere in Bezug auf den Einfluss der reflektierten Pulsdruckwelle herangezogen werden können. Deshalb können diese beiden Maxima auch sehr gut und mit Vorteil zur Anpassung der Filtercharakteristik, insbesondere der aktuellen Tiefpass-Grenzfrequenz, verwendet werden.

Gemäß einer weiteren günstigen Ausgestaltung wird als aktueller Wert der Tiefpass-Grenzfrequenz ein Frequenzwert des zweiten Amplitudenmaximums verwendet, wenn der Quotient des zweiten Amplitudenmaximums zu dem dritten Amplitudenmaximum höchstens gleich einem Quotientengrenzwert ist, wenn also insbes. gilt

$$\frac{2.Maximum}{3.Maximum} \leq Quotientengrenzwert \text{ , und an-}$$

dernfalls, wenn also insbesondere gilt

$$\frac{2.Maximum}{3.Maximum} > Quotientengrenzwert ,$$

3.Maximum als aktueller Wert der Tiefpass-Grenzfrequenz ein Frequenzwert des dritten Amplitudenmaximums verwendet, wobei der Quotientengrenzwert im Bereich zwischen 2,0 und 3,5, insbesondere zwischen 2,5 und 3,0, bevorzugt bei etwa 2,8 liegt. Es wurde erkannt, dass die aktuell herrschenden Gegebenheiten sehr gut abgebildet und berücksichtigt werden, wenn die Wahl der Tiefpass-Grenzfrequenz davon abhängig gemacht wird, wie der Quotient des zweiten und dritten Amplitudenmaximums zu dem genannten Quotientengrenzwert liegt. Je nach dem Ergebnis dieser Quotienten-Überprüfung wird als Tiefpass-Grenzfrequenz die Frequenz des zweiten oder des dritten Amplitudenmaximums gewählt, also die Frequenz, bei der das zweite bzw. das dritte Amplitudenmaximum liegt.

Gemäß einer weiteren günstigen Ausgestaltung wird das Verfahren während einer Kalibrierung eingesetzt. Während der Kalibrierung auftretende Messfehler sind besonders gravierend, da sie auch die später während des normalen Messbetriebs erfassten Messergebnisse negativ beeinflussen. Deshalb ist es günstig, während der Kalibrierung eine besonders hohe Messgenauigkeit zu erzielen und insbesondere die störenden Einflüsse der reflektierten Pulsdruckwelle auszuschließen oder zumindest weitest gehend zu reduzieren.

Gemäß einer weiteren günstigen Ausgestaltung wird der reflektierte Anteil der Pulsdruckwelle als ein Differenzsignal entsprechend einer Differenz des erfassten Pulsmesssignals und des korrigierten Pulsmesssignals ermittelt und insbesondere gesondert ausgewertet, vorzugsweise um insbesondere zusätzliche Informationen zur Geschwindigkeit der Pulsdruckwelle (= Pulswellengeschwindigkeit (PWG)), zur Pulsdruckwellenlaufzeit (Pulse Transit Time = PTT) zwischen dem Herzen und der Pulsmessstelle oder zu statischen und insbesondere zu dynamischen Eigenschaften des Herz- und Gefäßsystems des Patienten, wie z.B. dem Zustand und/oder dem Verhalten des Herz- und Gefäßsystems, zu gewinnen. Bei diesen statischen oder dynamischen Eigenschaften des Herz- und Gefäßsystems kann es sich z.B. um die Dehnbarkeit oder Elastizität (engl.: compliance) der Gefäße des Patienten oder um die Präejektionsperiode (engl.: pre-ejection period (PEP)) handeln. Diese zusätzlichen Informationen können dann vorteilhafterweise dazu verwendet werden, um insbesondere die Pulswellenanalyse zu verbessern, z.B. durch Kompensation der Präejektionsperiode (PEP). Insgesamt können anhand dieser zusätzlichen Informationen die Genauigkeit und Qualität der Messergebnisse weiter verbessert werden. Das Differenzsignal kann dabei entweder durch tatsächliche Bildung der Differenz des erfassten Pulsmesssignals und des korrigierten Pulsmesssignals im Zeitbereich oder aber z.B. auch durch Rücktransformation der bei der adaptiven Filterung zur Ermittlung des korrigierten Pulsmesssignals eigentlich im Frequenzbereich heraus gefilterten, d.h. verworfenen bzw. gelöschten, Frequenzanteile in den Zeitbereich ermittelt werden. Insbesondere können das Differenzsignal (als Maß für den reflektierten oder zum Herzen zurücklaufenden Anteil der Pulsdruckwelle) und das korrigierte Pulsmesssignal (als Maß für den vom Herzen weglaufenden Anteil der Pulsdruckwelle) dazu verwendet werden, die Pulswellengeschwindigkeit (PWG) und/oder die Pulsdruckwellenlaufzeit (PTT) alleine auf Basis des Pulsmesssignals, also insbesondere ohne Zuhilfenahme eines EKG-Messsignals, zu ermitteln. Dazu wird beispielsweise ein Zeitversatz zwischen dem vom Herzen weglaufenden Anteil der Pulsdruckwelle und dem zum Herzen zurücklaufenden Anteil der Pulsdruckwelle, also zwischen dem korrigierte Pulsmesssignal und dem Differenzsignal, ermittelt. Dies kann z.B. durch Bestimmung einer Zeitdifferenz zwischen markanten und insbesondere zueinander korrespondierenden Zeitpunkten innerhalb des korrigierten Pulsmesssignals und des Differenzsignals erfolgen. Als markanter Zeitpunkt kommt insbesondere ein solcher in Frage, zu dem das jeweilige Signal eine maximale Steigung, vorzugsweise eine maximale absolute Steigung, also entweder einen maximalen Anstieg oder einen maximalen Abfall, hat. Insbesondere unter zusätzlicher Berücksichtigung der bekannten Laufstrecken, insbesondere vom Herzen zur Pulsmessstelle, vom Herzen zur Reflektionsstelle sowie zwischen der Pulsmessstelle und der Reflektionsstelle, lassen sich dann die Pulswellengeschwindigkeit (PWG) und/oder die Pulsdruckwellenlaufzeit (PTT) ermitteln. Der reflektierte Anteil der Pulsdruckwelle hat insbesondere zumindest eine Reflektion an einer Extremität, beispielsweise an einer der Fingerspitzen, erfahren. Insbesondere nach einer erneuten dann herznahen Reflektion, beispielsweise an einem der oberen großen arteriellen Gefäße und/oder an der Herzklappe, kann es zu einer Überlagerung mit einem neuen vom Herzen gerade zu diesem Zeitpunkt hervorgerufenen und vom Herzen weglaufenden Anteil der Pulsdruckwelle kommen. Liegt die Pulsmessstelle insbesondere an einer der Fingerspitzen, hat der dort erfasste maßgebliche reflektierte Anteil der Pulsdruckwelle die Laufstrecke zwischen der Herzgegend und der Extremität, hier der Fingerspitze, also insbesondere dreimal durchlaufen. Diese Laufstrecke zwischen der Herzgegend und beispielsweise der Fingerspitze kann sehr gut und zumindest in guter Näherung bestimmt werden. Die vorstehend erläuterte vorteilhafte Ermittlung der Pulswellengeschwindigkeit (PWG) und/oder der Pulsdruckwellenlaufzeit (PTT) und/oder - darauf basierend - des systolischen oder diastolischen Blutdrucks alleine auf Basis des Pulsmesssignals, insbesondere unter Verwendung des daraus ermittelten korrigierten Pulsmesssignals und des ebenfalls daraus ermittelten Differenzsignals, aber vorzugsweise ohne Verwendung eines EKG-Messsignals, stellt auch für sich alleine genommen einen eigenen Erfindungsgegenstand dar. Dies gilt sowohl für das Ermittlungsverfahren an sich als auch für ein Gerät, in dem dieses Verfahren implementiert ist. Bei einem derartigen Verfahren gemäß dieser eigenständigen Erfindung handelt es sich um ein Verfahren zur Bestimmung einer Pulswellengeschwindigkeit (PWG) und/oder einer Pulsdruckwellenlaufzeit (PTT) und/oder eines systolischen oder diastolischen Blutdrucks, bei dem ein Pulsmesssignal einer Pulsdruckwelle, die sich ausgehend vom Herzen innerhalb der Blutgefäße ausbreitet, an einer Pulsmessstelle erfasst wird, aus dem erfassten Pulsmesssignal mittels Signalverarbeitung ein korrigiertes Pulsmesssignal erzeugt wird, ein einen reflektierten Anteil der Pulsdruckwelle symbolisierendes und insbesondere als Differenz zwischen dem Pulsmesssignal und dem korrigierten Pulsmesssignal bestimmtes Differenzsignal ermittelt wird, und die Pulsdruckwellenlaufzeit zwischen dem Herzen und der Pulsmessstelle aus dem korrigierten Pulsmesssignal und dem Differenzsignal ermittelt wird und auf Basis der Pulsdruckwellenlaufzeit insbesondere außerdem auch die Pulswellengeschwindigkeit oder der systolische oder diastolische Blutdruck ermittelt wird, wobei insbesondere die Laufstrecke der Pulsdruckwelle zwischen dem Herzen und der Pulsmessstelle berücksichtigt wird.

Gemäß einer weiteren günstigen Ausgestaltung wird anhand des korrigierten Pulsmesssignals mindestens einer der physiologischen Parameter aus der Gruppe von einem an der insbesondere herzfernen Pulsmessstelle herrschenden Blutdruck, einem zentralen Blutdruck, einem herznahen Plethysmogramm sowie statischen und insbesondere dynamischen Eigenschaften des Herz- und Gefäßsystems des Patienten, wie vorzugsweise der Dehnbarkeit oder Elastizität (engl.: compliance)) der Gefäße des Patienten und der Präejektionsperiode (engl.: pre-ejection period (PEP)), bestimmt. Damit kann also z.B. der Blutdruck insbesondere sowohl an einer herzfernen, z.B. an einer Extremität gelegenen, Pulsmessstelle als auch in Herznähe erfasst werden, und das vorteilhafterweise sogar kontinuierlich. Damit ist eine dynamische Abbildung der Gegebenheiten möglich. Eine derartige insbesondere kontinuierliche Erfassung des herznahen Blutdrucks ist mit anderen Verfahren bislang jedenfalls nicht ohne weiteres möglich. Auch ein vorzugsweise kontinuierliches herznahes Plethysmogramm lässt sich bestimmen. Insgesamt können anhand des Verfahrens also vorteilhafterweise viele physiologische Parameter ermittelt werden, von denen einige anderweitig nicht oder nur mit erheblich höherem Aufwand einer Erfassung zugänglich wären.

Eine weitere Aufgabe der Erfindung besteht darin, ein Gerät der eingangs bezeichneten Art mit einer gegenüber dem Stand der Technik verbesserten Erfassungsqualität anzugeben.

Zur Lösung der das Gerät betreffenden Aufgabe wird ein Gerät entsprechend den Merkmalen des Anspruchs 9 angegeben. Das erfindungsgemäße Gerät hat einen Pulssensor zur Erfassung eines Pulsmesssignals einer Pulsdruckwelle, die sich ausgehend vom Herzen innerhalb der Blutgefäße bis zu einer Pulsmessstelle ausbreitet, an der der Pulssensor angeordnet ist, sowie eine Auswerteeinheit zur Ermittlung eines korrigierten Pulsmesssignals mittels Signalverarbeitung aus dem erfassten Pulsmesssignal und zur Ermittlung des mindestens einen physiologischen Parameters anhand des korrigierten Pulsmesssignals, wobei die Auswerteeinheit dazu ausgelegt ist, zur Erzeugung des korrigierten Pulsmesssignals das erfasste Pulsmesssignal einer adaptiven Filterung mit einer sich dynamisch anpassenden Filtercharakteristik zu unterziehen, um den Einfluss eines reflektierten Anteils der Pulsdruckwelle zu kompensieren. Die Auswerteeinheit ist außerdem dazu ausgelegt, das erfasste Pulsmesssignal in jeweils einem Herzschlag zuzuordnende Messabschnitte zu zerlegen, aus jedem Messabschnitt mittels adaptiver Filterung einen korrigierten Abschnitt zu ermitteln und die so erzeugten korrigierten Abschnitte zu dem korrigierten Pulsmesssignal zusammenzusetzen.

Das erfindungsgemäße Gerät hat im Wesentlichen die gleichen bevorzugten Ausgestaltungen wie das erfindungsgemäße Verfahren. Außerdem bieten das erfindungsgemäße Gerät und seine bevorzugten Ausgestaltungen im Wesentlichen die gleichen Vorteile, die bereits im Zusammenhang mit dem erfindungsgemäßen Verfahren und dessen Varianten beschrieben worden sind. Die Auswerteeinheit kann dabei insbesondere sowohl Teil einer einzigen Baueinheit oder aber insbesondere auch auf zwei oder noch mehr Baueinheiten aufgeteilt sein.

Weitere Merkmale, Vorteile und Einzelheiten der Erfindung ergeben sich aus der nachfolgenden Beschreibung von Ausführungsbeispielen anhand der Zeichnung. Es zeigt:

Fig. 1     ein als Blockschaltbild dargestelltes Ausführungsbeispiel eines Blutdruckmessgeräts zur nichtinvasiven Bestimmung des Blutdrucks eines Patienten unter Verwendung eines korrigierten Pulsmesssignals,

Fig. 2     im Rahmen des Blutdruckmessgeräts gemäß Fig. 1 erfasste oder abgeleitete Signalverläufe,

Fig. 3     Signalverläufe des Pulsmesssignals bei verschiedenen Überlagerungen der originären und der reflektierten Pulsdruckwelle,

Fig. 4     ein Frequenzspektrum eines erfassten Pulsmesssignals und

Fig. 5     Signalverläufe des erfassten Pulsmesssignals, des korrigierten Pulsmesssignals und des Differenzsignals.

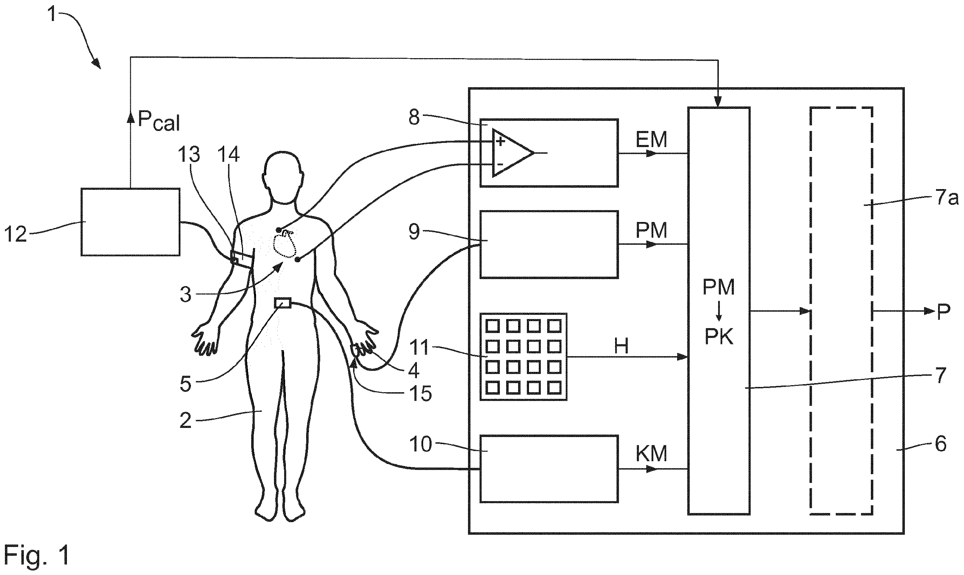

Einander entsprechende Teile sind in den Fig. 1 bis 5 mit denselben Bezugszeichen versehen. Auch Einzelheiten der im Folgenden näher erläuterten Ausführungsbeispiele können für sich genommen eine Erfindung darstellen oder Teil eines Erfindungsgegenstands sein.

In Fig. 1 ist als Beispiel für ein Gerät zur Erfassung eines physiologischen Parameters ein Blutdruckmessgerät 1 zur nichtinvasiven kontinuierlichen Bestimmung des (systolischen oder diastolischen) Blutdrucks P eines Patienten 2 gezeigt, wobei der Blutdruck P den zu erfassenden physiologischen Parameter darstellt. Der Aufbau und die grundsätzliche Funktionsweise eines solchen auf einer Auswertung der Pulswellenlaufzeit (Pulse Transit Time = PTT) basierenden Geräts sind in der DE 10 2005 014 048 B4 beschrieben.

Das Blutdruckmessgerät 1 enthält einen EKG-Sensor 3 mit mindestens zwei Aufnahmeelektroden, einen Pulssensor 4 insbesondere in Form eines Pulsoximeters oder eines photoplethysmographischen Sensors sowie einen optionalen Körperlagesensor 5 insbesondere in Form eines 3D-Beschleunigungssensors, die an eine Auswerteeinheit 6 angeschlossen sind. Die Auswerteeinheit 6 umfasst mehrere Komponenten. Neben einer ersten Berechnungseinheit 7 sowie einer optionalen (und deshalb in Fig. 1 nur in gestrichelter Linienführung eingetragenen) zweiten Berechnungseinheit 7a sind für jeden der angeschlossenen Sensoren spezifische Untereinheiten vorhanden, also eine EKG-Teileinheit 8, eine photoplethysmographische Teileinheit 9 sowie eine Körperlage-Teileinheit 10. Diese Komponenten der Auswerteeinheit 6 müssen nicht unbedingt physikalisch getrennt ausgebildet sein. Sie können auch als Unterprogramme einer auf einem Signal- oder Mikroprozessor in der Auswerteeinheit 6 ablaufenden Software realisiert sein. Ebenso ist es möglich, dass diese Komponenten in einer einzigen Baueinheit untergebracht oder aber auf zwei oder noch mehr Baueinheiten verteilt sind. Insbesondere können sich die erste Berechnungseinheit 7 und die optionale zweite Berechnungseinheit 7a in physikalisch voneinander getrennten Geräten befinden. Weiterhin umfasst die Auswerteeinheit 6 Eingabemittel 11, mittels derer Parameter, wie z.B. die Körpergröße H des Patienten 2, eingegeben werden können.

An die (ggf. auch mehrteilige) Auswerteeinheit 6 kann zumindest temporär eine Kalibrierungseinheit 12 mit einem herkömmlichen Blutdrucksensor 13 angeschlossen werden. Der Blutdrucksensor 13 ist im Ausführungsbeispiel als Riva-Rocci-Blutdrucksensor mit einer aufpumpbaren Armmanschette 14 ausgebildet. Ein während einer Kalibrierungsmessung mittels des Blutdrucksensors 13 und der Armmanschette 14 ermittelter Kalibrierungsblutdruckwert $P_{cal}$ wird an die Auswerteeinheit 6 weitergeleitet.

Bei dem Ausführungsbeispiel gemäß Fig. 1 ist der EKG-Sensor 3 herznah am Brustkorb des Patienten 2 angeordnet. Der Pulssensor 4 ist an einer Pulsmessstelle 15, bei dem Ausführungsbeispiel an einem Finger des Patienten 2, also insbesondere herzfern, angebracht. Eine andere Pulsmessstelle 15 wie z.B. an einem Ohr, einer Zehe oder einer Gliedmaße ist ebenso möglich. Außerdem kann der Pulssensor 4 anstatt als photoplethysmographischer Sensor oder als Pulsoximeter auch als Drucksensor oder als Ultraschallsensor ausgebildet sein.

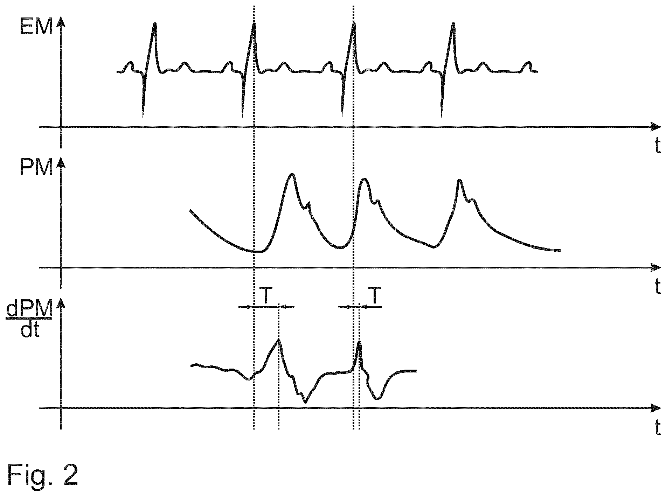

Die Funktionsweise des Blutdruckmessgeräts 1 während des Normalbetriebs ergibt sich aus den in Fig. 2 wiedergegebenen Diagrammen, in denen die Signalverläufe jeweils über der Zeit t aufgetragen sind. Die EKG-Teileinheit 8 erzeugt aus den von dem EKG-Sensor 3 erfassten Signalen ein elektrisches Messsignal EM (siehe oberes Diagramm von Fig. 2) eines Herzstroms, das zur Weiterverarbeitung in die Berechnungseinheit 7 eingespeist wird. Der Pulssensor 4 erfasst eine an der Pulsmessstelle 15 vorbeilaufende Pulsdruckwelle, die sich ausgehend vom Herzen des Patienten 2 innerhalb der Blutgefäße ausbreitet. Dementsprechend stellt die photoplethysmographische Teileinheit 9 auf Basis der mit dem Pulssensor 4 erfassten Signale der Berechnungseinheit 7 ein Pulsmesssignal PM (siehe mittleres Diagramm von Fig. 2) zur Verfügung. Die Körperlage-Teileinheit 10 liefert in Verbindung mit dem Körperlagesensor 5 ein (in Fig. 2 nicht mit dargestelltes) Körperlagesignal KM an die Berechnungseinheit 7. In der Berechnungseinheit 7 erfolgt die Verarbeitung insbesondere digital. Dementsprechend werden das elektrische Messsignal EM, das Pulsmesssignal PM und das Körperlagesignal KM vor ihrer Weiterverarbeitung insbesondere digitalisiert.

In der Auswerteeinheit 6, insbesondere in der ersten Berechnungseinheit 7 und der optionalen zweiten Berechnungseinheit 7a, wird aus dem elektrischen Messsignal EM und dem Pulsmesssignal PM eine Laufzeit T der Pulsdruckwelle zwischen dem Herzen des Patienten 2 und der Pulsmessstelle 15 ermittelt. Als Laufzeit T wird die Zeitdifferenz zwischen dem Zeitpunkt der sogenannten R-Zacke im elektrischen Messsignal EM und dem Zeitpunkt der maximalen Steigung im Pulsmesssignal PM verwendet. Zur leichteren Ermittlung des zuletzt genannten Zeitpunkts wird die zeitliche Ableitung des Pulsmesssignals PM gebildet (siehe unteres Diagramm von Fig. 2). Die gesuchte Laufzeit T lässt sich dann durch einen zeitlichen Vergleich der Maximalwerte im Messsignal EM und in der zeitlichen Ableitung des Pulsmesssignals PM bestimmen. In Fig. 2 sind für zwei aufeinanderfolgende Herzschlagzyklen die so bestimmten jeweiligen Laufzeiten T eingetragen. Die vorstehenden Ausführungen gelten insbesondere für die Ermittlung des systolischen Blutdrucks. Bei der grundsätzlich analog erfolgenden Ermittlung des diastolischen Blutdrucks wird als Laufzeit T insbesondere die Zeitdifferenz zwischen dem Zeitpunkt des Minimums im Pulsmesssignal PM und dem Zeitpunkt einer der zur Diastole korrespondierenden Zacken im elektrischen Messsignal EM verwendet.

Aus der ermittelten Laufzeit T wird in der Berechnungseinheit 7 mittels des in der DE 10 2005 014 048 B4 erläuterten Funktionszusammenhangs unter Berücksichtigung weiterer Parameter ein momentaner Blutdruck P errechnet.

Zur genauen Ermittlung des momentanen Werts des Blutdrucks P kommt es also mit entscheidend darauf an, den Zeitpunkt der maximalen Steigung im Pulsmesssignal PM möglichst exakt zu erfassen. Es hat sich gezeigt, dass dieser Zeitpunkt nicht in allen Konstellationen ohne Weiteres eindeutig ermittelt werden kann. Dies gilt insbesondere dann, wenn die originäre Pulsdruckwelle, also die vom Herzen ausgesendete Pulsdruckwelle, sich mit einer reflektierten oder rücklaufenden Pulsdruckwelle überlagert. Eine derartige rücklaufende Pulsdruckwelle kann sich aufgrund von Reflexionen an Übergangsbereichen von Gefäßstrukturen und/oder durch hydrodynamische Effekte bilden. Die Gestalt der reflektierten Pulsdruckwelle kann sich von der der originären Pulsdruckwelle in Abhängigkeit von den Gefäßeigenschaften des Patienten 2 unterscheiden.

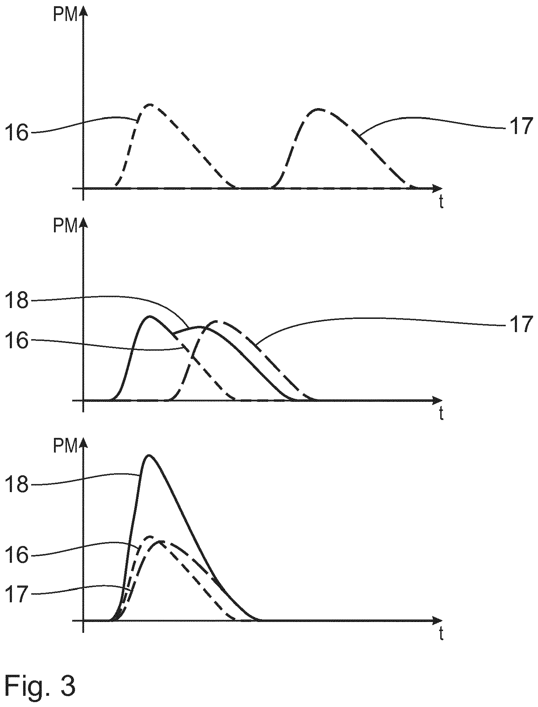

Es sind die durch die Signalverläufe gemäß Fig. 3 wiedergegebenen Konstellationen zu unterscheiden. Bei dem oberen Signalverlauf von Fig. 3 liegt zwischen der originären Pulsdruckwelle 16 (dargestellt in gestrichelter Linienführung) und der reflektierten Pulsdruckwelle 17 (dargestellt in gestrichelter Linienführung) ein zeitlicher Abstand, so dass beide Pulsdruckwellenanteile leicht voneinander zu unterscheiden und auch zu separieren sind. Bei der im mittleren Diagramm dargestellten Konstellation überlappen sich die originäre Pulsdruckwelle 16 und die reflektierte Pulsdruckwelle 17 (jeweils wiederum in gestrichelter Linienführung dargestellt) etwas, so dass sich für eine aus beiden Anteilen zusammensetzende Pulsdruckwelle 18 das in durchgezogener Linienführung dargestellte Pulsmesssignal PM ergibt. Bei der im unteren Diagramm von Fig. 3 wiedergegebenen Konstellation besteht eine weitreichende Überlappung der originären Pulsdruckwelle 16 und der reflektierten Pulsdruckwelle 17. Die erfassbare Pulsdruckwelle 18 enthält als einen Bestandteil die originäre Pulsdruckwelle 16 und als einen weiteren Bestandteil die reflektierte Pulsdruckwelle 17, wobei diese beiden Bestandteile zumindest auf den ersten Blick nicht mehr aus dem erfassbaren Pulsmesssignal PM der (kombinierten) Pulsdruckwelle 18 hervorgehen. Während in den ersten beiden Konstellationen in der Anstiegsflanke des resultierenden Pulsmesssignals PM der Zeitpunkt der maximalen Steigung noch sehr gut ermittelt werden kann, ist dies in der im unteren Diagramm gemäß Fig. 3 wiedergegebenen dritten Konstellation nicht mehr mit der gewünschten Eindeutigkeit möglich, so dass es hier zu Messfehlern kommen kann. Besonders gravierend sind die Auswirkungen auf die Messgenauigkeit, wenn eine Konstellation, wie im dritten Diagramm gemäß Fig. 3 wiedergegeben, während der Kalibrierung des Blutdruckmessgeräts 1 auftritt.

Um diese negativen Auswirkungen auf die Messgenauigkeit auszuschließen, umfasst das Blutdruckmessgerät 1 eine Kompensation des störenden Einflusses der reflektierten Pulsdruckwelle 17. Insbesondere ist die Auswerteeinheit 6 dazu ausgelegt, diese Kompensation durchzuführen. In der Auswerteeinheit 6 ist ein Korrekturalgorithmus implementiert, der die Zwei- oder Mehrdeutigkeit des Zeitpunkts des steilsten Anstiegs in der ersten ansteigenden Flanke des Pulsmesssignals PM beseitigt. Dieser Korrekturalgorithmus basiert auf der Erkenntnis, dass durch die Überlagerung der originären Pulsdruckwelle 16 mit der reflektierten Pulsdruckwelle 17 höhere Frequenzanteile generiert werden. Dementsprechend umfasst der Korrekturalgorithmus eine adaptive Frequenzfilterung, die abhängig von der aktuellen - und insbesondere vom Korrekturalgorithmus auch erkannten - Konstellation einen bestimmten höherfrequenten Anteil des Pulsmesssignals PM entfernt, so dass er für die weitere Signalauswertung, insbesondere bei der Ermittlung des Zeitpunkts des maximalen Anstiegs in der ersten Flanke des Pulsmesssignals PM, nicht berücksichtigt wird. Im Rahmen des Korrekturalgorithmus wird also ein korrigiertes Pulsmesssignals PK erzeugt, das im Wesentlichen nur Frequenzkomponenten umfasst, die mit der originären Pulsdruckwelle 16 unmittelbar korreliert sind.

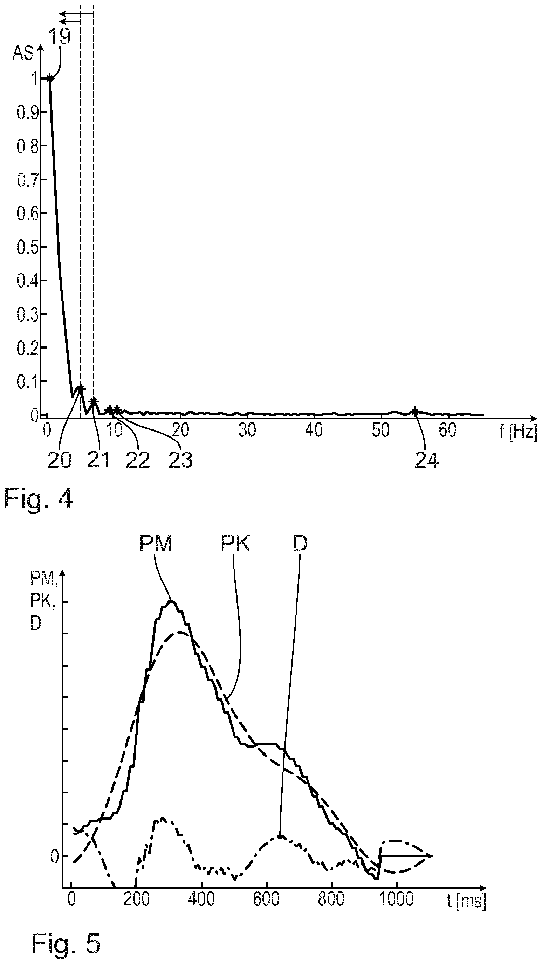

Bei dem Ausführungsbeispiel ist dieser Korrekturalgorithmus folgendermaßen realisiert. Aus dem ursprünglich erfassten Pulsmesssignal PM wird nach einer Digitalisierung des aufgenommenen Messsignals jeweils ein einem Herzschlag zuzuordnender Teilabschnitt extrahiert und der eigentlichen Signalkorrektur unterzogen. Der extrahierte Teilabschnitt wird beispielsweise mittels einer diskreten Fouriertransformation (DFT) in den Frequenzbereich transformiert. Zur Erreichung einer gewünschten Frequenzauflösung, beispielsweise von etwa 0,5 Hz, wird bei Bedarf der extrahierte Teilabschnitt des erfassten und digitalisierten Messsignals PM am Ende mit Nullen ergänzt. Das nach der Zeit-Frequenz-Transformation resultierende Frequenzsignal (= anfängliches Frequenzsignal) umfasst einen spektralen Amplitudenanteil sowie einen spektralen Phasenanteil. Zur weiteren Auswertung wird zunächst ein Frequenzspektrum des Amplitudenanteils ermittelt. Ein Beispiel eines dabei resultierenden Amplitudenspektrums AS ist in dem normierten und über der Frequenz f aufgetragenen Signalverlauf gemäß Fig. 4 wiedergegeben. Von diesem Amplitudenspektrum AS werden die lokalen Maxima detektiert. In der Abbildung gemäß Figur 4 sind die Maxima durch Sterne kenntlich gemacht und mit den Bezugszeichen 19 bis 24 gekennzeichnet.

Wie bereits erwähnt, stellt der Korrekturalgorithmus eine adaptive Filterung dar, die insbesondere eine an die aktuellen Gegebenheiten anpassbare Filtercharakteristik hat. Die Anpassung der Filtercharakteristik erfolgt anhand der detektierten Maxima 19 bis 24 des Amplitudenspektrums AS, insbesondere anhand des zweiten und dritten Maximums 20 bzw. 21. Dazu wird der Amplitudenwert des zweiten Maximums 20 durch den Amplitudenwert des dritten Maximums 21 dividiert. Danach wird überprüft, ob der so ermittelte Quotient über einem Schwellwert von etwa 2,8 liegt. Falls ja, werden alle Frequenzanteile bis einschließlich des Frequenzwerts des dritten Maximums 21 berücksichtigt. Andernfalls, also wenn der Quotient kleiner als der angegebene oder gleich dem angegebenen Schwellwert ist, werden nur Frequenzanteile bis einschließlich des Frequenzwerts des zweiten Maximums 20 berücksichtigt. Der Schwellwert kann auch als Quotientengrenzwert bezeichnet werden. Der Korrekturalgorithmus kann also als ein adaptives Tiefpassfilter mit einer variablen Tiefpass-Grenzfrequenz verstanden werden. Der für die Tiefpassfilterung aktuell verwendete Wert der Tiefpass-Grenzfrequenz richtet sich dabei nach den gerade herrschenden Gegebenheiten. Obwohl bei der Festlegung der aktuellen Filtercharakteristik auf das Amplitudenspektrum AS zurückgegriffen wird, wirkt die Tiefpassfilterung an sich sowohl auf den Amplitudenanteil als auch auf den Phasenanteil des in den Frequenzbereich transformierten Teilabschnitts des Pulsmesssignals PM.

Amplituden- und Phasenanteile mit einem Frequenzwert von höchstens dem des zweiten Maximums 20 des Amplitudenspektrum AS werden also immer berücksichtigt. Hintergrund ist die Erkenntnis, dass neben der Grundwelle auch die darunterliegenden Frequenzkomponenten, die durch Dämpfungen und Reflektionen im Gefäß entstehen, von entscheidender Bedeutung für die Form der originären Pulsdruckwelle 16 sind.

Der aktuelle Gefäßzustand unterliegt aber auch kurzfristigen Aktivierungen des autonomen Nervensystems. Diese äußern sich in einer Vasokonstriktion, die zu einer Versteifung der Gefäßwände führen. Um diese durch die Aktivität des autonomen Nervensystems zumindest mit hervorgerufenen Einflüsse angemessen zu berücksichtigen, ist es günstig, auch höher frequente Komponenten, nämlich insbesondere bis zum dritten Maximum 21 des Amplitudenspektrums AS, mit zu berücksichtigen. Es wurde erkannt, dass das oben angegebene Verhältnis der Amplituden des zweiten Maximums 20 zu der des dritten Maximums 21 einen guten Schätzwert für die Aktivität des autonomen Nervenssystems sowie für andere physiologische Gegebenheiten bildet. Insofern kann man also mit guter Näherung davon ausgehen, dass eine relevante Aktivität des autonomen Nervensystems vorliegt, wenn das Verhältnis über dem genannten Schwellwert liegt. In diesem Fall berücksichtigt der Korrekturalgorithmus, wie vorstehend ausgeführt, im Rahmen der adaptiven Filterung mehr Frequenzkomponenten.

Nachdem die Tiefpass-Grenzfrequenz der adaptiven Filterung entsprechend den vorstehenden Regelungen festgelegt worden ist, wird die Filterung durchgeführt. Hierbei werden alle Amplituden- und Phasenanteile, die bei einer höheren als der ermittelten Tiefpass-Grenzfrequenz liegen, gelöscht bzw. zu Null gesetzt. Das dabei frequenzgefilterte Restspektrum (= korrigiertes Frequenzsignal), welches sowohl Amplituden- als auch Phasenanteile umfasst, wird danach in den Zeitbereich zurück transformiert, beispielsweise mittels einer inversen Fouriertransformation, um so zu einem Teilabschnitt des korrigierten Pulsmesssignals PK zu gelangen. Durch Zusammensetzung der so ermittelten einzelnen jeweils einem Herzschlag zugeordneten korrigierten Teilabschnitte erhält man einen kontinuierlichen Verlauf des korrigierten Pulsmesssignals PK. Da die Ermittlung des korrigierten Pulsmesssignals PK mit einem gewissen Rechenaufwand verbunden ist, kann bei Bedarf die optionale zweite Berechnungseinheit 7a zum Einsatz kommen. Hierbei kann es sich insbesondere um einen leistungsstarken Rechner handeln. Grundsätzlich ist es aber auch möglich, dass alle Berechnungen zur Ermittlung des korrigierten Pulsmesssignals PK nur in einer einzigen Berechnungseinheit, nämlich in der ersten Berechnungseinheit 7, erfolgen.

In dem Diagramm gemäß Fig. 5 ist neben dem ursprünglich erfassten Pulsmesssignal PM auch das in der vorstehend beschriebenen Weise korrigierte Pulsmesssignal PK sowie das als Differenz zwischen dem ursprünglich erfassten Pulsmesssignal PM und dem korrigierten Pulsmesssignal PK gebildete Differenzsignal D über der Zeit t aufgetragen. Das ursprüngliche Pulsmesssignal PM ist in durchgezogener Linienführung, das korrigierte Pulsmesssignal PK in gestrichelter Linienführung und das Differenzsignal D in strichpunktierter Linienführung wiedergegeben. Aus den in Fig. 5 wiedergegebenen Signalverläufen ist deutlich zu entnehmen, dass das korrigierte Pulsmesssignal PK gegenüber dem ursprünglich erfassten Pulsmesssignal PM einen geglätteten Verlauf aufweist. Insbesondere zeigt die Anstiegsflanke einen monotonen Anstiegsverlauf, so dass sich auch der gesuchte Punkt mit dem maximalen Anstieg ohne Weiteres und vor allem eindeutig ermitteln lässt. Durch den beschriebenen Korrekturalgorithmus ist der Einfluss der reflektierten Pulsdruckwelle 17 also zumindest weitgehend ausgeglichen worden.

Die einzelnen Teilabschnitte des ursprünglich erfassten Pulsmesssignals PM, die jeweils einem Herzschlag zuzuordnen sind, werden in gleicher Weise dem Korrekturalgorithmus unterzogen. Die korrigierten Teilabschnitte werden dann zu einem Gesamtverlauf des korrigierten Pulsmesssignals PK zusammengesetzt.

Als Nebenprodukt erhält man das Differenzsignal D, das außer durch die bereits angesprochene im Zeitbereich erfolgende Differenzbildung zwischen dem ursprünglich erfassten Pulsmesssignal PM und dem korrigierten Pulsmesssignal PK alternativ auch durch eine Rücktransformation der bei der oben erwähnten adaptiven Filterung eigentlich gelöschten bzw. unberücksichtigt gelassenen Frequenzanteile vom Frequenz- in den Zeitbereich generiert werden kann. Das Differenzsignal D beschreibt die rücklaufende Pulsdruckwelle 17. Anhand des Differenzsignals D lassen sich weitere Analysen durchführen. So ist es möglich, beispielsweise anhand von Form und relativer Lage der reflektierten Pulsdruckwelle 17 zusätzliche Informationen über den Zustand des Gefäßsystems zu erhalten. Außerdem beschreibt auch das korrigierte Pulsmesssignal PK den Zustand des Gefäßsystems unmittelbarer als das ursprünglich erfasste Pulsmesssignal PM, das eine Überlagerung mit dem auf die reflektierte Pulsdruckwelle 17 zurückgehenden Anteil darstellt. Folglich kann neben dem Differenzsignal D zusätzlich oder alternativ auch das korrigierte Pulsmesssignal PK für weitere Analysen herangezogen werden.

Bei den weiteren Analysen der reflektierten Pulsdruckwelle 17, insbesondere anhand des Differenzsignals D, lassen sich zusätzliche Informationen, beispielsweise bezüglich der Pulswellengeschwindigkeit und bezüglich anderer Gefäßeigenschaften, wie z. B. der Dehnbarkeit oder Elastizität (= Compliance) der Gefäße, gewinnen. Außerdem können dynamische Parameter des Herz- und Gefäßsystems abgeschätzt werden, die dann ihrerseits zur weiteren Verbesserung der Pulswellenanalyse herangezogen werden können. Insbesondere lässt sich dann auch die Präejektionsperiode (= PEP) zumindest zu einem gewissen Umfang kompensieren.

Das korrigierte Pulsmesssignal PK gibt aufgrund der Kompensation der reflektierten Pulsdruckwelle 17 die ursprünglich vom Herzen erzeugten Pulsdruckwelle 16 deutlich realistischer wieder als das fern vom Herzen an einer Extremität, bei dem gezeigten Ausführungsbeispiel an einem Finger, erfasste Pulsmesssignal PM. Folglich lassen sich anhand des korrigierten Pulsmesssignals PK Aussagen zur Form der Pulsdruckwelle 16 in Herznähe treffen. In Herznähe kommt es nämlich zumindest anfangs noch nicht zu einer Überlagerung mit reflektierten Anteilen.

Außerdem erlaubt das korrigierte Pulsmesssignal PK eine verbesserte Abschätzung des zentralen, also in Herznähe herrschenden, Blutdrucks P. In Herznähe ist eine unmittelbare Erfassung des Blutdrucks P nicht oder zumindest nicht ohne erheblichen Aufwand möglich.

Das Blutdruckmessgerät 1 und insbesondere der in der Auswerteeinheit 6 implementierte Korrekturalgorithmus sind also sehr vorteilhaft. Das implementierte adaptive Filter passt seine Filtercharakteristik an die physiologischen Gegebenheiten des Patienten 2 und insbesondere dessen autonome Aktivierung dynamisch an. Durch die so vorgenommene adaptive Filterung wird zumindest eine erhebliche Abschwächung der Störeinflüsse der reflektierenden Pulsdruckwelle 17 erreicht, wodurch der Blutdruck P genauer ermittelt werden kann. Der Korrekturalgorithmus kann sowohl während einer Kalibrierung als auch während des eigentlichen Messbetriebs des Blutdruckmessgeräts 1 zum Einsatz kommen.

Wie vorstehend erläutert, bietet das Kompensationsverfahren aber auch zahlreiche andere Möglichkeiten zur Erfassung verschiedener physiologischer Parameter, wie beispielsweise der Geschwindigkeit der Pulsdruckwelle, statischer und/oder dynamischer Eigenschaften des Herz- und Gefäßsystems, inklusive der Dehnbarkeit (= Compliance) der Gefäße und der Präejektionsperiode. Deshalb lässt sich der beschriebene Korrekturalgorithmus nicht nur im Zusammenhang mit einer Blutdruckmessung mit Vorteil anwenden, sondern auch bei der Erfassung weiterer physiologischer Parameter. Das vorstehend beschriebene Blutdruckmessgerät 1 ist insofern nur beispielhaft zu verstehen. Der Korrekturalgorithmus lässt sich analog auf andere Erfassungsverfahren und -geräte übertragen. Auch bei diesen weiteren Anwendungen kommen die vorstehend beschriebenen Vorteile in gleicher oder ähnlicher Weise zum Tragen.

**Patentansprüche**

1. Verfahren zur Bestimmung mindestens eines physiologischen Parameters (P) eines Patienten (2), bei dem

> a) ein Pulsmesssignal (PM) einer Pulsdruckwelle (18), die sich ausgehend vom Herzen innerhalb der Blutgefäße ausbreitet, an einer Pulsmessstelle (15) mittels eines Pulssensors (4) erfasst wird,
> b) aus dem erfassten Pulsmesssignal (PM) mittels Signalverarbeitung ein korrigiertes Pulsmesssignal (PK) erzeugt wird,
> c) der mindestens eine physiologische Parameter (P) anhand des korrigierten Pulsmesssignals (PK) ermittelt wird, wobei
> d) zur Erzeugung des korrigierten Pulsmesssignals (PK) das erfasste Pulsmesssignal (PM) einer Filterung unterzogen wird, um den Einfluss eines reflektierten Anteils (17) der Pulsdruckwelle (18) zu kompensieren,
> **dadurch gekennzeichnet, dass** es sich bei der Filterung um eine adaptive Filterung mit einer sich dynamisch anpassenden Filtercharakteristik handelt
> wobei
> e) das erfasste Pulsmesssignal (PM) in jeweils einem Herzschlag zuzuordnende Messabschnitte zerlegt wird, aus jedem Messabschnitt mittels adaptiver Filterung ein korrigierter Abschnitt ermittelt wird und die so erzeugten korrigierten Abschnitte zu dem korrigierten Pulsmesssignal (PK) zusammengesetzt werden.

2. Verfahren nach Anspruch 1, **dadurch gekennzeichnet, dass** der betreffende Messabschnitt des erfassten Pulsmesssignals (PM) mittels Transformation in den Frequenzbereich in ein anfängliches Frequenzsignal überführt wird, das anfängliche Frequenzsignal zur Anpassung der Filtercharakteristik herangezogen wird und dann der adaptiven Filterung mit der angepassten Filtercharakteristik unterzogen wird, wobei ein korrigiertes Frequenzsignal gebildet wird, das mittels Rücktransformation in den Zeitbereich in den korrigierten Abschnitt überführt wird.

3. Verfahren nach einem der vorhergehenden Ansprüche, **dadurch gekennzeichnet, dass** die adaptive Filterung als adaptive Tiefpassfilterung mit einer variablen Tiefpass-Grenzfrequenz ausgeführt wird.

4. Verfahren nach Anspruch 2, **dadurch gekennzeichnet, dass** die adaptive Filterung als adaptive Tiefpassfilterung mit einer variablen Tiefpass-Grenzfrequenz ausgeführt wird, von dem anfänglichen Frequenzsignal die Amplitudenmaxima (19 - 24) bestimmt werden, und aus dem Quotienten des zweiten Amplitudenmaximums (20) zu dem dritten Amplitudenmaximum (21) der aktuelle Wert der Tiefpass-Grenzfrequenz ermittelt wird, um die Filtercharakteristik anzupassen.

5. Verfahren nach Anspruch 4, **dadurch gekennzeichnet, dass** als aktueller Wert der Tiefpass-Grenzfrequenz ein Frequenzwert des zweiten Amplitudenmaximums (20) verwendet wird, wenn der Quotient des zweiten Amplitudenmaximums (20) zu dem dritten Amplitudenmaximum (21) höchstens gleich einem Quotientengrenzwert ist, und andernfalls als aktueller Wert der Tiefpass-Grenzfrequenz ein Frequenzwert des dritten Amplitudenmaximums (21) verwendet wird, wobei der Quotientengrenzwert im Bereich zwischen 2,0 und 3,5, insbesondere zwischen 2,5 und 3,0, bevorzugt bei 2,8 liegt.

6. Verfahren nach einem der vorhergehenden Ansprüche, **dadurch gekennzeichnet, dass** es während einer Kalibrierung eingesetzt wird.

7. Verfahren nach einem der vorhergehenden Ansprüche, **dadurch gekennzeichnet, dass** der reflektierte Anteil (17) der Pulsdruckwelle (18) als ein Differenzsignal (D) entsprechend einer Differenz des erfassten Pulsmesssignals (PM) und des korrigierten Pulsmesssignals (PK) ermittelt wird und insbesondere gesondert ausgewertet wird, vorzugsweise um Informationen zur Geschwindigkeit der Pulsdruckwelle, zur Pulsdruckwellenlaufzeit zwischen dem Herzen und der Pulsmessstelle (15) oder zu statischen und insbesondere zu dynamischen Eigenschaften des Herz- und Gefäßsystems des Patienten, wie vorzugsweise zu der Dehnbarkeit der Gefäße des Patienten oder zu der Präejektionsperiode, zu gewinnen.

8. Verfahren nach einem der vorhergehenden Ansprüche, **dadurch gekennzeichnet, dass** anhand des korrigierten Pulsmesssignals (PK) mindestens einer der physiologischen Parameter aus der Gruppe von einem an der Pulsmessstelle herrschenden Blutdruck (P), einem zentralen Blutdruck, einem herznahen Plethysmogramm sowie statischen und insbesondere dynamischen Eigenschaften des Herz- und Gefäßsystems des Patienten, wie vorzugsweise der Dehnbarkeit der Gefäße des Patienten und der Präejektionsperiode, bestimmt wird.

9. Gerät zur Bestimmung mindestens eines physiologischen Parameters (P) eines Patienten (2) umfassend

a) einen Pulssensor (4) zur Erfassung eines Pulsmesssignals (PM) einer Pulsdruckwelle (18), die sich ausgehend vom Herzen innerhalb der Blutgefäße bis zu einer Pulsmessstelle (15) ausbreitet, an der der Pulssensor (4) angeordnet ist,
b) eine Auswerteeinheit (6) zur Ermittlung eines korrigierten Pulsmesssignals (PK) mittels Signalverarbeitung aus dem erfassten Pulsmesssignal (PM) und zur Ermittlung des mindestens einen physiologischen Parameters (P) anhand des korrigierten Pulsmesssignals (PK), und
c) die Auswerteeinheit (6) dazu ausgelegt ist, zur Erzeugung des korrigierten Pulsmesssignals (PK) das erfasste Pulsmesssignal (PM) einer Filterung zu unterziehen, um den Einfluss eines reflektierten Anteils (17) der Pulsdruckwelle (18) zu kompensieren, **dadurch gekennzeichnet, dass** es sich bei der Filterung um eine adaptive Filterung mit einer sich dynamisch anpassenden Filtercharakteristik handelt, wobei
d) die Auswerteeinheit (6) dazu ausgelegt ist, das erfasste Pulsmesssignal (PM) in jeweils einem Herzschlag zuzuordnende Messabschnitte zu zerlegen, aus jedem Messabschnitt mittels adaptiver Filterung einen korrigierten Abschnitt zu ermitteln und die so erzeugten korrigierten Abschnitte zu dem korrigierten Pulsmesssignal (PK) zusammenzusetzen.

10. Gerät nach Anspruch 9, **dadurch gekennzeichnet, dass** die Auswerteeinheit (6) dazu ausgelegt ist, den betreffende Messabschnitt des erfassten Pulsmesssignals (PM) mittels Transformation in den Frequenzbereich in ein anfängliches Frequenzsignal zu überführen, das anfängliche Frequenzsignal zur Anpassung der Filtercharakteristik heranzuziehen und dann der adaptiven Filterung mit der angepassten Filtercharakteristik zu unterziehen, wobei sich ein korrigiertes Frequenzsignal ergibt, und die Auswerteeinheit (6) weiterhin dazu ausgelegt ist, das korrigierte Frequenzsignal mittels Rücktransformation in den Zeitbereich in den korrigierten Abschnitt zu überführen.

11. Gerät nach einem der Ansprüche 9 und 10, **dadurch gekennzeichnet, dass** die Auswerteeinheit (6) dazu ausgelegt ist, die adaptive Filterung als adaptive Tiefpassfilterung mit einer variablen Tiefpass-Grenzfrequenz auszuführen.

12. Gerät nach Anspruch 10, **dadurch gekennzeichnet, dass** die Auswerteeinheit (6) dazu ausgelegt ist, die adaptive Filterung als adaptive Tiefpassfilterung mit einer variablen Tiefpass-Grenzfrequenz auszuführen, von dem anfänglichen Frequenzsignal die Amplitudenmaxima (19 - 24) zu bestimmen, und aus dem Quotienten des zweiten Amplitudenmaximums (20) zu dem dritten Amplitudenmaximum (21) den aktuelle Wert den Tiefpass-Grenzfrequenz zu ermitteln, um die Filtercharakteristik anzupassen.

13. Gerät nach Anspruch 12, **dadurch gekennzeichnet, dass** die Auswerteeinheit (6) dazu ausgelegt ist, als aktuellen Wert der Tiefpass-Grenzfrequenz einen Frequenzwert des zweiten Amplitudenmaximums (20) zu verwenden, wenn der Quotient des zweiten Amplitudenmaximums (20) zu dem dritten Amplitudenmaximum (21) höchstens gleich einem Quotientengrenzwert ist, und andernfalls als aktuellen Wert der Tiefpass-Grenzfrequenz einen Frequenzwert des dritten Amplitudenmaximums (21) zu verwenden, wobei der Quotientengrenzwert im Bereich zwischen 2,0 und 3,5, insbesondere zwischen 2,5 und 3,0, bevorzugt bei 2,8 liegt.

14. Gerät nach einem der Ansprüche 9 bis 13, **dadurch gekennzeichnet, dass** die Auswerteeinheit (6) dazu ausgelegt ist, die Ermittlung des korrigierten Pulsmesssignals (PK) und die auf dem korrigierten Pulsmesssignal (PK) basierende Ermittlung des mindestens einen physiologischen Parameters (P) während einer Kalibrierung des Geräts durchzuführen.

15. Gerät nach einem der Ansprüche 9 bis 14, **dadurch gekennzeichnet, dass** die Auswerteeinheit (6) dazu ausgelegt ist, den reflektierten Anteil (17) der Pulsdruckwelle (18) als ein Differenzsignal (D) entsprechend einer Differenz des erfassten Pulsmesssignals (PM) und des korrigierten Pulsmesssignals (PK) zu ermitteln und insbesondere gesondert auszuwerten, vorzugsweise um Informationen zur Geschwindigkeit der Pulsdruckwelle, zur Pulsdruckwellenlaufzeit zwischen dem Herzen und der Pulsmessstelle (15) oder zu statischen und insbesondere zu dynamischen Eigenschaften des Herz- und Gefäßsystems des Patienten, wie vorzugsweise zu der Dehnbarkeit der Gefäße des Patienten oder zu der Präejektionsperiode, zu gewinnen.

16. Gerät nach einem der Ansprüche 9 bis 15, **dadurch gekennzeichnet, dass** die Auswerteeinheit (6) dazu ausgelegt ist, anhand des korrigierten Pulsmesssignals (PK) mindestens einen der physiologischen Parameter aus der Gruppe von einem an der Pulsmessstelle herrschenden Blutdruck (P), einem zentralen Blutdruck, einem herznahen Plethysmogramm sowie statischen und insbesondere dynamischen Eigenschaften des Herz- und Gefäßsystems des Patienten, wie vorzugsweise der Dehnbarkeit der Gefäße des Patienten und der Präejektionsperiode, zu bestimmen.

**Claims**

1. A method for determining at least one physiological parameter (P) of a patient (2), in which

   a) a pulse measurement signal (PM) of a pulse pressure wave (18) propagating within the blood vessels and emanating from the heart is acquired at a pulse measurement point (15) by means of a pulse sensor (4),
   b) a corrected pulse measurement signal (PK) is produced from the acquired pulse measurement signal (PM) by means of signal processing,
   c) the at least one physiological parameter (P) is ascertained on the basis of the corrected pulse measurement signal (PK), wherein
   the acquired pulse measurement signal (PM), for the purposes of producing the corrected pulse measurement signal (PK), is subjected to filtering in order to compensate the influence of a reflected component (17) of the pulse pressure wave (18),
   **characterized in that** the filtering is an adaptive filtering with a dynamically adapting filter characteristic, wherein
   e) according to the acquired pulse measurement signal (PM) is decomposed into measurement sections which can respectively be assigned to a heartbeat, a corrected section is ascertained from each measurement section by means of adaptive filtering, and the corrected sections thus produced are composed to form the corrected pulse measurement signal (PK).

2. Method according to claim 1, **characterized in that** the relevant measurement section of the acquired pulse measurement signal (PM) is converted into an initial frequency signal by means of a transformation into the frequency domain, the initial frequency signal is used for adapting the filter characteristic and then subjected to adaptive filtering with the adapted filter characteristic, wherein a corrected frequency signal is formed, said corrected frequency signal being converted into the corrected section by means of a back transformation into the time domain.

3. Method according to any one of the preceding claims, **characterized in that** the adaptive filtering is carried out as adaptive low-pass filtering with a variable low-pass cutoff frequency.

4. Method according to claim 2, **characterized in that** the adaptive filtering is carried out as adaptive low-pass filtering with a variable low-pass cutoff frequency, the amplitude maxima (19-24) of the initial frequency signal are determined, and the current value of the low-pass cutoff frequency is ascertained from the quotient of the second amplitude maximum (20) to the third amplitude maximum (21) in order to adapt the filter characteristic.

5. Method according to claim 4, **characterized in that** a frequency value of the second amplitude maximum (20) is used as current value of the low-pass cutoff frequency if the quotient of the second amplitude maximum (20) to the third amplitude maximum (21) at most equals a quotient threshold, and otherwise a frequency value of the third amplitude maximum (21) is used as current value of the low-pass cutoff frequency, wherein the quotient threshold lies in the range between 2.0 and 3.5, in particular between 2.5 and 3.0, preferably at 2.8.

6. Method according to any one of the preceding claims, **characterized in that** it is used during a calibration.

7. Method according to any one of the preceding claims, **characterized in that** the reflected component (17) of the pulse pressure wave (18) is ascertained as a difference signal (D) corresponding to a difference between the acquired pulse measurement signal (PM) and the corrected pulse measurement signal (PK) and, in particular, evaluated separately, preferably in order to obtain information in respect of the speed of the pulse pressure wave, in respect of the pulse pressure wave transit time between the heart and the pulse measurement point (15) or in respect of static and, in particular, dynamic properties of the cardiac system and vessel system of the patient like, preferably, in respect of the compliance of the vessels of the patient or in respect of the pre-ejection period.

8. Method according to any one of the preceding claims, **characterized in that** the corrected pulse measurement signal (PK) is used to determine at least one of the physiological parameters of the group containing a blood pressure (P) prevailing at the pulse measurement point, a central blood pressure, a plethysmogram in the proximity of the heart, and static and, in particular, dynamic properties of the cardiac system and vessel system of the patient like, preferably, the compliance of the vessels of the patient and the pre-ejection period.

9. Device for determining at least one physiological parameter (P) of a patient (2), comprising

    a) a pulse sensor (4) for acquiring a pulse measurement signal (PM) of a pulse pressure wave (18) which, emanating from the heart, propagates within the blood vessels up to a pulse measurement point (15) at which the pulse sensor (4) is arranged, and
    b) an evaluation unit (6) for ascertaining a corrected pulse measurement signal (PK) from the acquired pulse measurement signal (PM) by means of signal processing and for ascertaining the at least one physiological parameter (P) on the basis of the corrected pulse measurement signal (PK), andc) the evaluation unit (6) is configured, for the purposes of producing the corrected pulse measurement signal (PK), to subject the acquired pulse measurement signal (PM) to filtering in order to compensate the influence of a reflected component (17) of the pulse pressure wave (18)
    **characterized in that** the filtering is an adaptive filtering with a dynamically adapting filter characteristic, wherein
    d) the evaluation unit (6) is configured to decompose the acquired pulse measurement signal (PM) into measurement sections which can respectively be assigned to a heartbeat, ascertain a corrected section from each measurement section by means of adaptive filtering, and compose the corrected sections thus produced to form the corrected pulse measurement signal (PK).

10. Device according to claim 9, **characterized in that** the evaluation unit (6) is configured to convert the relevant measurement section of the acquired pulse measurement signal (PM) into an initial frequency signal by means of a transformation into the frequency domain, use the initial frequency signal for adapting the filter characteristic and then subject said initial frequency signal to adaptive filtering with the adapted filter characteristic, wherein a corrected frequency signal is formed, and the evaluation unit (6) is further configured to convert the corrected frequency signal into the corrected section by means of a back transformation into the time domain.

11. Device according to any one of claims 9 and 10, **characterized in that** the evaluation unit (6) is configured to carry out the adaptive filtering as adaptive low-pass filtering with a variable low-pass cutoff frequency.

12. The device according to claim 10, **characterized in that** the evaluation unit (6) is configured to carry out the adaptive filtering as adaptive low-pass filtering with a variable low-pass cutoff frequency, determine the amplitude maxima (19-24) of the initial frequency signal, and ascertain the current value of the low-pass cutoff frequency from the quotient of the second amplitude maximum (20) to the third amplitude maximum (21) in order to adapt the filter characteristic.

**13.** The device according to claim 12, **characterized in that** the evaluation unit (6) is configured to use a frequency value of the second amplitude maximum (20) as current value of the low-pass cutoff frequency if the quotient of the second amplitude maximum (20) to the third amplitude maximum (21) at most equals a quotient threshold, and otherwise use a frequency value of the third amplitude maximum (21) as current value of the low-pass cutoff frequency, wherein the quotient threshold lies in the range between 2.0 and 3.5, in particular between 2.5 and 3.0, preferably at 2.8.

**14.** The device according to any one of claims 9 to 13, **characterized in that** the evaluation unit (6) is configured to perform ascertaining the corrected pulse measurement signal (PK) and ascertaining the at least one physiological parameter (P) on the basis of the corrected pulse measurement signal (PK) during a calibration of the device.

**15.** The device according to any one of claims 9 to 14, **characterized in that** the evaluation unit (6) is configured to ascertain the reflected component (17) of the pulse pressure wave (18) as a difference signal (D) corresponding to a difference between the acquired pulse measurement signal (PM) and the corrected pulse measurement signal (PK) and, in particular, to evaluate said reflected component separately, preferably in order to obtain information in respect of the speed of the pulse pressure wave, in respect of the pulse pressure wave transit time between the heart and the pulse measurement point (15) or in respect of static and, in particular, dynamic properties of the cardiac system and vessel system of the patient like, preferably, in respect of the compliance of the vessels of the patient or in respect of the pre-ejection period.

**16.** The device according to any one of claims 9 to 15, **characterized in that** the evaluation unit (6) is configured to use the corrected pulse measurement signal (PK) to determine at least one of the physiological parameters of the group containing a blood pressure (P) prevailing at the pulse measurement point, a central blood pressure, a plethysmogram in the proximity of the heart, and static and, in particular, dynamic properties of the cardiac system and vessel system of the patient like, preferably, the compliance of the vessels of the patient and the pre-ejection period.

**Revendications**

**1.** Procédé de détermination d'au moins un paramètre physiologique (P) d'un patient (2), par lequel

    a) un signal de mesure du pouls (PM) d'une onde de pression de pulsation (18), qui se propage depuis le coeur au sein des vaisseaux sanguins, est saisi au niveau d'un emplacement de mesure de pulsation (15) au moyen d'un capteur de pulsation (4),
b) un signal de mesure du pouls corrigé (PK) est produit à partir du signal de mesure du pouls (PM) saisi au moyen d'un traitement de signal,
c) le au moins un paramètre physiologique (P) est établi à l'aide du signal de mesure du pouls corrigé (PK), dans lequel
d) pour produire le signal de mesure du pouls corrigé (PK), le signal de mesure du pouls (PM) saisi est soumis à un filtrage, en vue de contrebalancer l'effet d'une partie réfléchie (17) de l'onde de pression de pulsation (18),
**caractérisé en ce que** le filtrage est un filtrage adaptatif avec une caractéristique de filtre s'adaptant de façon dynamique
dans lequel
e) le signal de mesure du pouls (PM) saisi est décomposé en des tronçons de mesure attribuables à une pulsation du coeur respective, à partir de chaque tronçon de mesure est établi au moyen d'un filtrage adaptatif un tronçon corrigé, et les tronçons corrigés ainsi produits sont assemblés pour former le signal de mesure du pouls corrigé (PK).

**2.** Procédé selon la revendication 1, **caractérisé en ce que** le tronçon de mesure concerné du signal de mesure du pouls (PM) saisi est transformé en un signal de fréquence initial au moyen d'une transformation dans la gamme de fréquences, le signal de fréquence initial est repris pour ajuster la caractéristique de filtre, puis est soumis au filtrage adaptatif avec la caractéristique de filtre adaptée, dans lequel un signal de fréquence corrigé est formé qui est transformé en tronçon corrigé au moyen d'une transformation inverse dans la plage de temporisation.

**3.** Procédé selon l'une quelconque des revendications précédentes, **caractérisé en ce que** le filtrage adaptatif est réalisé en tant que filtrage adaptatif passe-bas avec une fréquence de coupure passe-bas variable.

**4.** Procédé selon la revendication 2, **caractérisé en ce que** le filtrage adaptatif est réalisé en tant que filtrage adaptatif passe-bas avec une fréquence de coupure passe-bas variable, les amplitudes maximales (19-24) sont déterminées à partir du signal de fréquence initial, et la valeur en cours de la fréquence de coupure passe-bas est établie à partir du ratio de la deuxième amplitude maximale (20) sur la troisième amplitude maximale (21), en vue d'adapter la caractéristique de filtre.

**5.** Procédé selon la revendication 4, **caractérisé en ce qu'**en tant que valeur en cours de la fréquence de coupure passe-bas est utilisée une valeur de fréquence de la deuxième amplitude maximale (20) lorsque le ratio de la deuxième amplitude maximale (20) sur la troisième amplitude maximale (21) est au plus égal à une valeur seuil de ratio, et à défaut en tant que valeur en cours de la fréquence de coupure passe-bas est utilisée une valeur de fréquence de la troisième amplitude maximale (21), dans lequel la valeur seuil de ratio se situe dans la plage allant de 2,0 à 3,5, en particulier de 2,5 à 3,0, de préférence est égale à 2,8.

**6.** Procédé selon l'une quelconque des revendications précédentes, **caractérisé en ce qu'**il est engagé durant un étalonnage.

**7.** Procédé selon l'une quelconque des revendications précédentes, **caractérisé en ce que** la partie réfléchie (17) de l'onde de pression de pulsation (18) est établie en tant que signal différentiel (D) correspondant à une différence entre le signal de mesure du pouls (PM) saisi et le signal de mesure du pouls corrigé (PK) et est exploité séparément, de préférence en vue d'obtenir des informations sur la vitesse de l'onde de pression de pulsation, sur la durée de l'onde de pression de pulsation entre le coeur et l'emplacement de mesure de pulsation (15) ou sur les propriétés statiques et en particulier dynamiques du système cardio-vasculaire du patient, ainsi que de préférence sur l'extensibilité des vaisseaux du patient ou sur la période de pré-éjection.

**8.** Procédé selon l'une quelconque des revendications précédentes, **caractérisé en ce qu'**à l'aide du signal de mesure du pouls corrigé (PK) est déterminé au moins un des paramètres physiologiques du groupe formé par une pression artérielle (P) régissant l'emplacement de mesure de pulsation, une pression artérielle centrale, un pléthysmogramme à proximité du coeur ainsi que des propriétés statiques et en particulier dynamiques du système cardio-vasculaire du patient, ainsi que de préférence l'extensibilité des vaisseaux du patient et la période de pré-éjection.

**9.** Appareil destiné à déterminer au moins un paramètre physiologique (P) d'un patient (2) comprenant

a) un capteur de pulsation (4) pour saisir un signal de mesure du pouls (PM) d'une onde de pression de pulsation (18), qui se propage depuis le coeur au sein des vaisseaux sanguins jusqu'à un emplacement de mesure de pulsation (15), contre lequel est agencé le capteur de pulsation (4),
b) une unité d'exploitation (6) pour établir un signal de mesure du pouls corrigé (PK) au moyen d'un traitement de signal à partir du signal de mesure du pouls (PM) saisi et pour établir un paramètre physiologique (P) à l'aide du signal de mesure du pouls corrigé (PK), et
c) l'unité d'exploitation (6) est conçue pour, en vue de produire le signal de mesure du pouls corrigé (PK), soumettre le signal de mesure du pouls (PM) saisi à un filtrage en vue de contrebalancer l'effet d'une partie réfléchie (17) de l'onde de pression de pulsation (18),
**caractérisé en ce que** le filtrage est un filtrage adaptatif avec une caractéristique de filtre s'adaptant de façon dynamique
dans lequel
d) l'unité d'exploitation (6) est conçue pour décomposer le signal de mesure du pouls (PM) en des tronçons de mesure attribuables à une pulsation du coeur respective, établir à partir de chaque tronçon de mesure au moyen d'un filtrage adaptatif un tronçon corrigé, et assembler les tronçons corrigés ainsi produits pour former le signal de mesure du pouls corrigé (PK).

**10.** Appareil selon la revendication 9, **caractérisé en ce que** l'unité d'exploitation (6) est conçue pour transformer le tronçon de mesure concerné du signal de mesure du pouls (PM) saisi en un signal de fréquence initial au moyen d'une transformation dans la gamme de fréquences, reprendre le signal de fréquence initial pour ajuster la caractéristique de filtre, puis soumettre au filtrage adaptatif avec la caractéristique de filtre adaptée, dans lequel un signal de fréquence corrigé en résulte, et l'unité d'exploitation (6) est en outre conçue pour transformer le signal de fréquence corrigé en tronçon corrigé au moyen d'une transformation inverse dans la plage de temporisation.

**11.** Appareil selon l'une des revendications 9 et 10, **caractérisé en ce que** l'unité d'exploitation (6) est conçue pour réaliser le filtrage adaptatif en tant que filtrage adaptatif passe-bas avec une fréquence de coupure passe-bas variable.

**12.** Appareil selon la revendication 10, **caractérisé en ce que** l'unité d'exploitation (6) est conçue pour réaliser le filtrage adaptatif en tant que filtrage adaptatif passe-bas avec une fréquence de coupure passe-bas variable, déterminer les amplitudes maximales (19-24) à partir du signal de fréquence initial, et établir la valeur en cours de la fréquence de coupure passe-bas à partir du ratio de la deuxième amplitude maximale (20) sur la troisième amplitude maximale (21), en vue d'adapter la caractéristique de filtre.

**13.** Appareil selon la revendication 12, **caractérisé en ce que** l'unité d'exploitation (6) est conçue pour utiliser en tant que valeur en cours de la fréquence de coupure passe-bas une valeur de fréquence de la deuxième amplitude maximale (20) lorsque le ratio de la deuxième amplitude maximale (20) sur la troisième amplitude maximale (21) est au plus égal à une valeur seuil de ratio, et utiliser à défaut en tant que valeur en cours de la fréquence de coupure passe-bas une valeur de fréquence de la troisième amplitude maximale (21), dans lequel la valeur seuil de ratio se situe dans la plage allant de 2,0 à 3,5, en particulier de 2,5 à 3,0, de préférence est égale à 2,8.

14. Appareil selon l'une quelconque des revendications 9 à 13, **caractérisé en ce que** l'unité d'exploitation (6) est conçue pour réaliser l'établissement du signal de mesure du pouls corrigé (PK) et l'établissement fondé sur le signal de mesure du pouls corrigé (PK) du au moins un paramètre physiologique (P) pendant un étalonnage de l'appareil.

15. Appareil selon l'une quelconque des revendications 9 à 14, **caractérisé en ce que** l'unité d'exploitation (6) est conçue pour établir la partie réfléchie (17) de l'onde de pression de pulsation (18) en tant que signal différentiel (D) correspondant à une différence entre le signal de mesure du pouls (PM) saisi et le signal de mesure du pouls corrigé (PK) et l'exploiter séparément, de préférence en vue d'obtenir des informations sur la vitesse de l'onde de pression de pulsation, sur la durée de l'onde de pression de pulsation entre le coeur et l'emplacement de mesure de pulsation (15) ou sur les propriétés statiques et en particulier dynamiques du système cardio-vasculaire du patient, ainsi que de préférence sur l'extensibilité des vaisseaux du patient ou sur la période de pré-éjection.

16. Appareil selon l'une quelconque des revendications 9 à 15, **caractérisé en ce que** l'unité d'exploitation (6) est conçue pour déterminer à l'aide du signal de mesure du pouls corrigé (PK) au moins un des paramètres physiologiques du groupe formé par une pression artérielle (P) régissant l'emplacement de mesure de pulsation, une pression artérielle centrale, un pléthysmogramme à proximité du coeur ainsi que des propriétés statiques et en particulier dynamiques du système cardio-vasculaire du patient, ainsi que de préférence l'extensibilité des vaisseaux du patient et la période de pré-éjection.

Fig. 1

Fig. 2

Fig. 3

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102005014048 B4
- DE 102007024072 A1
- DE 68925988 T2
- DE 60130395 T2
- DE 602004000513 T2
- DE 102006022120 A1
- DE 19829544 C1
- EP 2491856 A1
- US 20140288445 A1
- DE 69835843 T2
- US 2004171944 A1
- US 20030036685 A1